# EUROPÄISCHE PATENTSCHRIFT

(11) **EP 3 494 257 B1**
(45) Veröffentlichungstag und Bekanntmachung des Hinweises auf die Patenterteilung: **17.06.2020**
(21) Anmeldenummer: 17746468.2
(22) Anmeldetag: 31.07.2017
(51) Int. Cl.: D21F 7/00, D21G 9/00, G01N 33/34

(54) **SENSORÜBERWACHUNG**
SENSOR MONITORING
SURVEILLANCE PAR CAPTEUR

(30) Priorität: 05.08.2016 DE 102016214512
(43) Veröffentlichungstag der Anmeldung: 12.06.2019
(73) Patentinhaber: Voith Patent GmbH, 89522 Heidenheim (DE)
(72) Erfinder: BRITZ, Herbert, 88276 Berg (DE); VOIRON, Sylvain, 88368 Bergatreute (DE)
(74) Vertreter: Voith Patent GmbH - Patentabteilung
(86) Internationale Anmeldenummer: PCT/EP2017/069293
(87) Internationale Veröffentlichungsnummer: WO 2018/024663

(56) Entgegenhaltungen:
- EP-A1- 2 947 426
- DE-A1- 10 253 822
- DE-B3-102005 041 178
- GB-A- 2 497 804

## Beschreibung

Die Erfindung betrifft ein Verfahren zur Überwachung eines Sensors, welcher in einem Prozess zur Herstellung und/oder Veredlung einer Papier-, Karton,- Tissue- oder einer anderen Faserstoffbahn oder zur Bildung und/oder Behandlung einer zur Herstellung der Faserstoffbahn geeigneten Faserstoffsuspension an einer Messstelle angeordnet ist um einen Parameter der Faserstoffsuspension oder der Faserstoffbahn zu messen und an eine Steuer- oder Regeleinheit des Prozesses oder eines Teilprozesses davon zu übermitteln.

Die Erfindung betrifft ebenso ein Verfahren zur Überwachung mehrerer Sensoren, welche in einem Prozess zur Herstellung und/oder Veredlung einer Papier-, Karton,-Tissue- oder einer anderen Faserstoffbahn und/oder zur Bildung und/oder Behandlung einer zur Herstellung der Faserstoffbahn geeigneten Faserstoffsuspension an unterschiedlichen Messstellen angeordnet sind um den gleichen Parameter der Faserstoffsuspension und/oder der Faserstoffbahn zu messen und an eine Steuer- oder Regeleinheit des Prozesses oder eines Teilprozesses davon zu übermitteln.

Verfahren und Anlagen zur Herstellung einer Faserstoffbahn, insbesondere zur Herstellung einer Papier-, Tissue- oder Kartonbahn, sind bekannt und umfassen üblicherweise eine Stoffaufbereitung, in welcher aus einem Fasern enthaltenden Rohstoff, wie Altpapier und/oder Zellstoff, eine Faserstoffsuspension hergestellt wird, und eine Papiermaschine, in welcher die aus der Stoffaufbereitung stammende Faserstoffsuspension zu Papier oder einer anderen Faserstoffbahn verarbeitet wird.

Zur Herstellung der Faserstoffsuspension aus dem Fasern enthaltenden Rohstoff wird der Rohstoff in einem, auch Pulper genannten Stofflöser mit Wasser vermischt und aufgelöst, bevor die so hergestellte Faserstoffsuspension während der anschließenden Prozessschritte sukzessive bis zu dem Endprodukt unter Wasserentzug konzentriert wird.

Bei Anlagen zur Papierherstellung handelt es sich um technisch aufwändige Großanlagen, die neben der eigentlichen Papiermaschine mit Nassteil, Trockenteil, Kalander, Roller und zugehörigen Peripherieeinheiten vorgeschaltete Einheiten der Stoffaufbereitung wie zum Beispiel Flotation, Sortierung, Dispergierung, Bleiche, Mahlung sowie nachgeschaltete Systeme wie Rollenwickeleinheiten sowie Rollenschneidmaschinen, die Rollenverpackung und den Rollentransport umfassen. Dazu gehören die jeweils erforderlichen elektrischen Antriebe, Pumpen, Vakuumanlagen, Chemikalienaufbereitung, Druckluftversorgung und/oder dergleichen.

Bekanntlich sind bei der Herstellung oder Behandlung einer Faserstoffsuspension bzw. einer Faserstoffbahn eine Vielzahl von Parametern zu beachten, um allen Anforderungen an die geforderte Qualität gerecht werden zu können. Daher haben derartige Maschinen eine Vielzahl von separaten Regelkreisen innerhalb der Stoffaufbereitung und innerhalb der Papiermaschine oder sogar über beide Einheiten hinweg.

Die Regelkreise benötigen dabei Messwerte für unterschiedliche Parameter, welche oft online von Sensoren entlang der Maschine geliefert werden. Auf Grund der, insbesondere bezüglich Hitze und Schmutz sehr anspruchsvollen Betriebsbedingungen müssen diese Sensoren regelmäßig überprüft, neu kalibriert und gegebenenfalls ausgetauscht werden. Hierzu werden die Sensorergebnisse meist auch mit parallel gewonnenen Labor-Messergebnissen vorzugsweise in regelmäßigen Abständen verglichen, wie dies beispielsweise in der DE 102 53 822 A1 ausgeführt ist.

Insgesamt ist diese Sensorüberwachung sehr zeit- und kostenaufwendig.

Die Aufgabe der Erfindung ist es daher die Sensorüberwachung zu vereinfachen.

Erfindungsgemäß wurde die Aufgabe bei der Überwachung nur eines Sensors dadurch gelöst, dass eine Überwachungseinheit auf der Basis eines Modells des Prozesses oder wenigstens eines Teilprozesses davon den Parameterwert an einer Messstelle berechnet und mit dem von dem Sensor dieser Messstelle übermittelten Messwert vergleicht und bei Überschreiten einer Maximalabweichung zwischen dem berechneten Parameterwert und dem übermittelten Messwert auf einen Sensorfehler, insbesondere einen Messfehler dieses Sensor an dieser Messstelle schließt.

Im Normalbetrieb stellen die vom Sensor übermittelten Messwerte lediglich eine Feinabstimmung der vom Modell berechneten Parameterwerte dar.
Weichen die Messwerte jedoch zu stark, d.h. über die vorgegebene Maximalabweichung hinaus davon ab, so ist eine neue Kalibrierung erforderlich oder ein Defekt des Sensors wahrscheinlich, weshalb ein Fehler des Sensors angezeigt wird.

Für eine Übergangszeit bis zur neuen Kalibrierung oder zum Wechsel des Sensors oder eventuell auch der Bestimmung einer anderen Fehlerquelle kann es vorteilhaft sein, dass bei einem Sensorfehler anstelle des Messwertes dieses Sensors der berechnete Parameterwert für diese Messstelle an die Steuer- oder Regeleinheit übermittelt wird.
Je nach Art des Sensors oder der Umweltbedingungen, beispielsweise bei einer zu befürchtenden, allmählichen Verschmutzung des Messfensters, kann es allerdings ebenso von Vorteil sein, dass bei einem Sensorfehler ein, um einen vorgegebenen Korrekturwert veränderter Messwert dieses Sensors an die Steuer- oder Regeleinheit übermittelt wird.
Beide Maßnahmen ermöglichen den Betrieb zumindest über eine bestimmte Zeitspanne auch bei einem Sensorfehler. Der Sensorwechsel kann, falls erforderlich, so wesentlich günstiger bei einem geplanten Servicetermin erfolgen.

Wird ein bestimmter Parameter von mehreren Sensoren erfasst, so ist es bei deren Überwachung erfindungswesentlich, dass eine Überwachungseinheit auf der Basis eines Modells des Prozesses oder wenigstens eines Teilprozesses davon den Differenzwert zwischen den Parameterwerten an jeweils zwei Messstellen berechnet und mit dem Differenzwert zwischen den, von den Sensoren der entsprechenden Messstellen übermittelten Messwerten vergleicht und bei Überschreiten einer Maximalabweichung zwischen den berechneten und den ermittelten Differenzwerten auf einen Sensorfehler, insbesondere einen Messfehler des entsprechenden Sensors an zumindest einer dieser beiden Messstellen schließt.

Bei der Überprüfung der Sensoren eines Parameters können jeweils beliebige Paare von Sensoren ausgewählt werden. Es kann aber ebenso vorteilhaft sein, bestimmte Paare von Sensoren auszuwählen, deren Messwerte gemäß dem Prozess oder Teilprozess stark korrelieren sollten, was die Fehlerauswertung vereinfacht.

Wird bei einem Paar von Sensoren ein Fehler festgestellt, so kann auch hier die Zeit bis zur neuen Kalibrierung oder zum Wechsel der Sensoren oder der Feststellung eines anderen Fehlers dadurch überbrückt werden, dass anstelle der Messwerte der beiden Sensoren, zwischen denen eine Überschreitung der Maximalabweichung festgestellt wird, die berechneten Parameterwerte für beide Messstellen an die Steuer- oder Regeleinheit übermittelt werden.

Zur Eingrenzung des Fehlers auf einen der beiden überwachten Sensoren kann es hilfreich sein, wenn die Überwachungseinheit bei einem Sensorfehler zumindest eine der beiden betroffenen Messstellen mit jeweils wenigstens einer weiteren Messstelle des Parameters hinsichtlich der Differenzwerte zwischen den berechneten Parameterwerten und den übermittelten Messwerten vergleicht.
Es dürfte höchst unwahrscheinlich sein, dass bei einer Messstelle, bei der in Bezug auf zumindest zwei Messstellen ein Sensorfehler erkannt wird, der Sensor dieser Messstelle keinen Defekt aufweist, weshalb anstelle des Messwertes dieses Sensors der berechnete Parameterwert für diese Messstelle an die Steuer- oder Regeleinheit übermittelt wird.
Andererseits kann auf einen funktionierenden Sensor bei einer Messstelle, bei der in Bezug auf nur eine von mehreren Messstellen ein Sensorfehler erkannt wird, geschlossen werden, weshalb der Messwert des Sensors dieser Messstelle an die Steuer- oder Regeleinheit übermittelt wird.

Wegen der großen Bedeutung für die Qualität, aber auch der relativ verbreiteten online-Messung eignet sich die Erfindung insbesondere für Sensoren deren zu erfassender Parameter die Weiße oder der Aschegehalt der Faserstoffsuspension und/oder der Faserstoffbahn ist.

Unabhängig davon werden wesentliche Parameter der Faserstoffsuspension und damit auch der daraus hergestellten Faserstoffbahn in der Stoffaufbereitung geprägt, weshalb der von den Sensoren überwachte und mit der Steuer- oder Regeleinheit gekoppelte Prozess bzw. Teilprozess die Bildung und/oder Behandlung einer Faserstoffsuspension umfassen sollte.
Auf Grund der großen Bedeutung für mehrere Parameter wie Weiße und Aschegehalt ist dabei vorteilhaft, wenn der Teilprozess wenigstens eine Flotationsstufe beinhaltet, wobei zumindest vor und/oder nach einer, vorzugsweise allen Flotationsstufen eine Messstelle vorhanden sein sollte.
Im Interesse einer möglichst genauen Regelung bei möglichst geringer Totzeit sollte wenigstens einer, vorzugsweise allen Flotationsstufen ein eigener Regelkreis zugeordnet sein. Eine Flotationsstufe kann dabei durchaus auch mehrere Flotationsbehälter umfassen.

Die Bereitstellung des berechneten Differenzwertes gestaltet sich hierbei relativ einfach, wenn der Regelkreis einer Flotationsstufe mit einer davor und danach angeordneten Messstelle als Basis für das Modell dieses Teilprozesses fungiert und den Differenzwert zwischen den berechneten Parameterwerten dieser Messstellen liefert.

Hinsichtlich der Wahl der zu überwachenden Paare von Sensoren kann die Qualität der Überwachung wesentlich gesteigert werden, wenn zwei Sensoren über den Differenzwert überwacht werden, zwischen deren Messstellen keine den Parameter veränderten Teilprozesse liegen. Dabei ist vom Modell allerdings die Verweilzeit der Faserstoffsuspension zwischen den beiden Messstellen zu berücksichtigen, welche beispielsweise durch die Zwischenlagerung in Stapeltürmen beeinflusst wird.

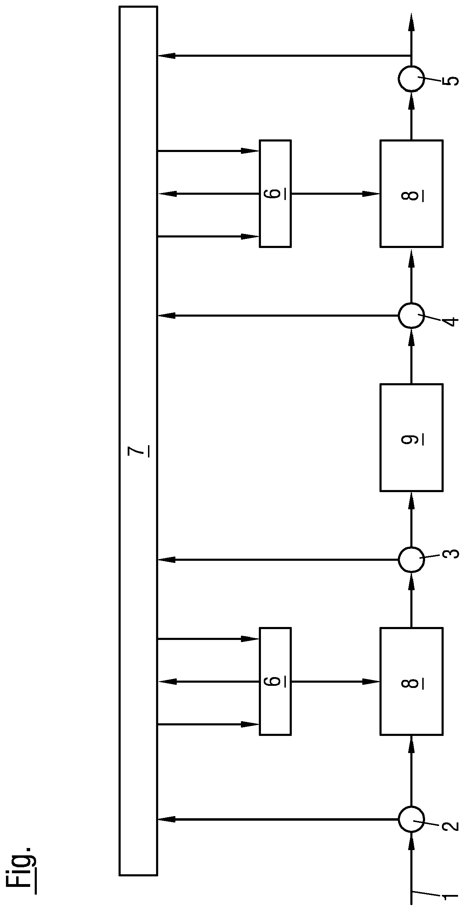

Allgemein sollte das Modell auf Erfahrungswerte insbesondere anderer Anlagen und/oder Daten der Inbetriebnahme und/oder eine Datenhistorie der Anlage zurückgreifen.
Darüber hinaus ist es von Vorteil, wenn das Modell als selbstlernendes System ausgelegt ist.
Nachfolgend soll die Erfindung an einem Ausführungsbeispiel näher erläutert werden. In der beigefügten Zeichnung zeigt die Figur ein Anlagenschema eines Abschnittes einer Stoffaufbereitung zur Herstellung einer Faserstoffsuspension zumindest teilweise aus Altpapier.

Die gesamte Anlage zur Herstellung einer Papierbahn besteht im Wesentlichen aus einer Stoffaufbereitung, einer Papiermaschine sowie einem Konstantteil zur Übergabe der Faserstoffsuspension von der Stoffaufbereitung an die Papiermaschine.
Es versteht sich, dass die Stoffaufbereitung und die Papiermaschine jeweils eine Vielzahl von Verfahrensschritten umfassen.
So beginnt die Papiermaschine in der Regel mit einem Stoffauflauf mit Nasspartie zur Blattbildung, woran sich eine Pressenpartie zur Entwässerung und eine Trockenpartie zur Trocknung der Faserstoffbahn anschließt. Je nach Art der herzustellenden Faserstoffbahn können dann noch Streich- und/oder Glättvorrichtungen folgen. Das fertig bearbeitete und geglättete Papier wird anschließend zu einer Rolle aufgewickelt.

Bei der Aufbereitung der Altpapiere in der Stoffaufbereitung finden in der Regel eine Anzahl verschiedener Verfahrensschritte statt, z.B. muss angelieferter Papierstoff in Wasser suspendiert, sortiert, flotiert, gemahlen, dispergiert und gereinigt werden. Diese Prozesse sind aber allgemein bekannt. Zumeist werden noch diverse Chemikalien bzw. Hilfsstoffe hinzugegeben. Am Ende der Stoffaufbereitung wird die Faserstoffsuspension in einem oder mehreren Stapeltürmen gesammelt.

Die in der Figur dargestellten Teilprozesse der Stoffaufbereitung umfassen zwei Flotationsstufen 8, die selbst wiederum aus mehreren Flotationsbehälter bestehen können. Jeder Flotationsstufe 8 ist dabei zur Bildung eines eigenen Regelkreises eine Regeleinheit 6 zugeordnet.
Zwischen den beiden Flotationsstufen 8 durchläuft die Faserstoffsuspension 1 einen oder mehrere Stapeltürme 9 zur Zwischenlagerung.
Vor und nach jeder Flotationsstufe 8 gibt es je eine Messstelle 2,3,4,5 mit einem Sensor zur Bestimmung des Aschegehaltes der Faserstoffsuspension 1. Dieser wichtige Parameter der Faserstoffsuspension 1 wird an eine Überwachungseinheit 7 übermittelt, die die Messwerte auf ihre Plausibilität prüft.
Diese Überwachungseinheit 7 arbeitet auf der Basis eines Modells des jeweiligen Teilprozesses und kann schnell offensichtliche Sensorfehler, insbesondere Messfehler erkennen. So kann der Aschegehalt der Faserstoffsuspension 1 in Strömungsrichtung der Stoffaufbereitung beispielsweise nicht ansteigen. Wird dies dennoch angezeigt, so liegt ein Sensorfehler vor.
Außerdem ist zu beachten, dass der Aschegehalt der Faserstoffsuspension 1 an den beiden Messstellen 3,4 vor und nach dem Stapelturm 9 unter Berücksichtigung der Verweilzeit, die beispielsweise zwischen 40 und 60 Minuten liegen kann, übereinstimmen sollte, da sich der Aschegehalt bei diesem Teilprozess nicht ändern kann.

Darüber hinaus berechnet die Überwachungseinheit 7 den Differenzwert zwischen den Aschegehaltswerten an zwei Messstellen 2,3,4,5 und vergleicht diesen mit dem Differenzwert zwischen den, von den Sensoren der entsprechenden Messstellen 2,3,4,5 übermittelten Messwerten.
Gibt es einen Unterschied zwischen dem berechneten und dem gemessenen Differenzwert und übersteigt dieser Unterschied eine vorgegebene Maximalabweichung so wird auf einen Sensorfehler an zumindest einer dieser beiden Messstellen 2,3,4,5 geschlossen.

Als eine mögliche Folge werden dann anstelle der Messwerte der beiden Sensoren, zwischen denen eine Überschreitung der Maximalabweichung festgestellt wird, die berechneten Aschegehaltswerte für beide Messstellen 2,3,4,5 an die Regeleinheit 6 übermittelt.

Alternativ kann die Überwachungseinheit 7 aber auch versuchen den Sensorfehler einzugrenzen und beide Messstellen 2,3,4,5, bei denen ein Sensorfehler festgestellt wurde, mit jeweils wenigstens einer weiteren Messstelle 2,3,4,5 des Aschegehaltes hinsichtlich der Differenzwerte zwischen den berechneten Parameterwerten und den übermittelten Messwerten vergleichen.
Wird bei diesem Test bei einer Messstelle 2,3,4,5 in Bezug auf zwei Messstellen 2,3,4,5 ein Sensorfehler erkannt, so ist die Wahrscheinlichkeit eines Kalibrierungsbedarfes oder Defektes bei diesem Sensor sehr groß. Daher wird anstelle des Messwertes dieses wahrscheinlich defekten Sensors der berechnete Parameterwert für diese Messstelle 2,3,4,5 an die Regeleinheit 6 übermittelt.
Ist die Abweichung bezüglich der beiden Messstellen 2,3,4,5 identisch, so kann die Überwachungseinheit 7 auch einen entsprechenden Korrekturwert bilden und den gemessen Parameterwert damit beaufschlagen und an die Regeleinheit 6 weitergeben. Sinnvoll ist dies insbesondere dann, wenn mit dieser Sensorbeeinträchtigung infolge Verschmutzung gerechnet werden kann.

Erkennt die Überwachungseinheit 7 jedoch bei einer Messstelle 2,3,4,5 in Bezug auf nur eine von mehreren Messstellen 2,3,4,5 einen Sensorfehler, so kann ein Sensorfehler nicht eindeutig diagnostiziert werden, so dass der Messwert des Sensors dieser Messstelle 2,3,4,5 an die Regeleinheit 6 übermittelt wird.
Eine neue Kalibrierung oder ein Wechsel des Sensors bei festgestelltem Sensorfehler ist so nicht zwingend sofort notwendig und kann bei geplanten Serviceterminen erfolgen, was die Wartungskosten der Anlage erheblich senkt.

Dabei bildet der Regelkreis der jeweiligen Flotationsstufen 8 mit einer davor und einer danach angeordneten Messstelle 2,3,4,5 die Basis für das Modell dieses Teilprozesses. Ausgehend von dem gemessenen Aschegehalt vor der Flotationsstufe 8 und dem Sollwert für den Aschegehalt der Faserstoffsuspension 1 nach dieser Flotationsstufe 8 werden die Stellgrößen der Flotation, wie Belüftungsintensität, Füllstand, Schaumwehrstellung usw. in bekannter Weise eingestellt.

Dementsprechend kann die Differenz zwischen dem gemessenen Aschegehalt vor der Flotationsstufe 8 und dem Sollwert nach der Flotationsstufe 8 als berechneter Differenzwert an die Überwachungseinheit 7 übergeben werden.

Die Überwachungseinheit 7 arbeitet auf der Basis mehrerer selbstlernender Modelle der entsprechenden Teilprozesse.
Die Anfangsdaten werden hierzu von Erfahrungswerten und/oder der Datenhistorie der Anlage gebildet und von dem selbstlernenden Modell während der Inbetriebnahme der Anlage optimiert.

## Patentansprüche

1. Verfahren zur Überwachung eines Sensors, welcher in einem Prozess zur Herstellung und/oder Veredlung einer Papier-, Karton,- Tissue- oder einer anderen Faserstoffbahn oder zur Bildung und/oder Behandlung einer zur Herstellung der Faserstoffbahn geeigneten Faserstoffsuspension (1) an einer Messstelle (2,3,4,5) angeordnet ist um einen Parameter der Faserstoffsuspension (1) oder der Faserstoffbahn zu messen und an eine Steuer- oder Regeleinheit (6) des Prozesses oder eines Teilprozesses davon zu übermitteln, **dadurch gekennzeichnet, dass** eine Überwachungseinheit (7) auf der Basis eines Modells des Prozesses oder wenigstens eines Teilprozesses davon den Parameterwert an einer Messstelle (2,3,4,5) berechnet und mit dem von dem Sensor dieser Messstelle (2,3,4,5) übermittelten Messwert vergleicht und bei Überschreiten einer Maximalabweichung zwischen dem berechneten Parameterwert und dem übermittelten Messwert auf einen Sensorfehler an dieser Messstelle (2,3,4,5) schließt.

2. Verfahren nach Anspruch 1, **dadurch gekennzeichnet, dass** bei einem Sensorfehler anstelle des Messwertes dieses Sensors der berechnete Parameterwert für diese Messstelle (2,3,4,5) an die Steuer- oder Regeleinheit (6) übermittelt wird.

3. Verfahren nach Anspruch 1, **dadurch gekennzeichnet, dass** bei einem Sensorfehler ein, um einen vorgegebenen Korrekturwert veränderter Messwert dieses Sensors an die Steuer- oder Regeleinheit (6) übermittelt wird.

4. Verfahren zur Überwachung mehrerer Sensoren, welche in einem Prozess zur Herstellung und/oder Veredlung einer Papier-, Karton,- Tissue- oder einer anderen Faserstoffbahn und/oder zur Bildung und/oder Behandlung einer zur Herstellung der Faserstoffbahn geeigneten Faserstoffsuspension (1) an unterschiedlichen Messstellen (2,3,4,5) angeordnet sind um den gleichen Parameter der Faserstoffsuspension (1) und/oder der Faserstoffbahn zu messen und an eine Steuer- oder Regeleinheit (6) des Prozesses oder eines Teilprozesses davon zu übermitteln, **dadurch gekennzeichnet, dass** eine Überwachungseinheit (7) auf der Basis eines Modells des Prozesses oder wenigstens eines Teilprozesses davon den Differenzwert zwischen den Parameterwerten an zwei Messstellen (2,3,4,5) berechnet und mit dem Differenzwert zwischen den, von den Sensoren der entsprechenden Messstellen (2,3,4,5) übermittelten Messwerten vergleicht und bei Überschreiten einer Maximalabweichung zwischen den berechneten und den ermittelten Differenzwerten auf einen Sensorfehler an zumindest einer dieser beiden Messstellen (2,3,4,5) schließt.

5. Verfahren nach Anspruch 4, **dadurch gekennzeichnet, dass** bei einem Sensorfehler anstelle der Messwerte der beiden Sensoren, zwischen denen eine Überschreitung der Maximalabweichung festgestellt wird, die berechneten Parameterwerte für beide Messstellen (2,3,4,5) an die Steuer- oder Regeleinheit (6) übermittelt werden.

6. Verfahren nach Anspruch 4, **dadurch gekennzeichnet, dass** die Überwachungseinheit (7) bei einem Sensorfehler zumindest eine der beiden betroffenen Messstellen (2,3,4,5) mit jeweils wenigstens einer weiteren Messstelle (2,3,4,5) des Parameters hinsichtlich der Differenzwerte zwischen den berechneten Parameterwerten und den übermittelten Messwerten vergleicht.

7. Verfahren nach Anspruch 6, **dadurch gekennzeichnet, dass** bei einer Messstelle (2,3,4,5), bei der in Bezug auf zumindest zwei Messstellen (2,3,4,5) ein Sensorfehler erkannt wird, anstelle des Messwertes dieses Sensors der berechnete Parameterwert für diese Messstelle (2,3,4,5) an die Steuer- oder Regeleinheit (6) übermittelt wird.

8. Verfahren nach Anspruch 6 oder 7, **dadurch gekennzeichnet, dass** bei einer Messstelle (2,3,4,5), bei der in Bezug auf nur eine von mehreren Messstellen (2,3,4,5) ein Sensorfehler erkannt wird, der Messwert des Sensors dieser Messstelle (2,3,4,5) an die Steuer- oder Regeleinheit (6) übermittelt wird.

9. Verfahren nach einem der vorhergehenden Ansprüche, **dadurch gekennzeichnet, dass** der Parameter von der Weiße oder dem Aschegehalt der Faserstoffsuspension (1) und/oder der Faserstoffbahn gebildet wird.

10. Verfahren nach einem der vorhergehenden Ansprüche, **dadurch gekennzeichnet, dass** der Prozess die Bildung und/oder Behandlung einer Faserstoffsuspension (1) umfasst.

11. Verfahren nach Anspruch 9 oder 10, **dadurch gekennzeichnet, dass** der Teilprozess wenigstens eine Flotationsstufe (8) beinhaltet.

12. Verfahren nach Anspruch 11, **dadurch gekennzeichnet, dass** zumindest vor und/oder nach einer, vorzugsweise allen Flotationsstufen (8) eine Messstelle (2,3,4,5) vorhanden ist.

13. Verfahren nach Anspruch 11 oder 12, **dadurch gekennzeichnet, dass** wenigstens einer, vorzugsweise allen Flotationsstufen (8) ein eigener Regelkreis zugeordnet ist.

14. Verfahren nach Anspruch 13, **dadurch gekennzeichnet, dass** der Regelkreis einer Flotationsstufe (8) mit einer davor und danach angeordneten Messstelle (2,3,4,5) als Basis für das Modell dieses Teilprozesses fungiert und den Differenzwert zwischen den berechneten Parameterwerten dieser Messstellen (2,3,4,5) liefert.

15. Verfahren nach einem der Ansprüche 10 bis 14, **dadurch gekennzeichnet, dass** in Fällen, bei denen zwischen zwei Messstellen (2,3,4,5) keine den Parameter veränderten Teilprozesse liegen, das Modell die Verweilzeit der Faserstoffsuspension (1) zwischen den beiden Messstellen (2,3,4,5) berücksichtigt.

16. Verfahren nach einem der vorhergehenden Ansprüche, **dadurch gekennzeichnet, dass** das Modell auf Erfahrungswerten insbesondere anderer Anlagen und/oder Daten der Inbetriebnahme und/oder der Historie der Anlage basiert.

17. Verfahren nach einem der vorhergehenden Ansprüche, **dadurch gekennzeichnet, dass** das Modell als selbstlernendes System ausgelegt ist.

## Claims

1. Method for monitoring a sensor which is arranged at a measurement location (2, 3, 4, 5) in a process for producing and/or finishing a paper, cardboard, tissue or other fibrous web and for forming and/or treating a fibrous suspension (1) suitable for producing the fibrous web, in order to measure a parameter of the fibrous suspension (1) or of the fibrous web and to transmit it to a control or regulating unit (6) of the process or a subprocess thereof, **characterized in that** a monitoring unit (7) calculates the parameter value at a measurement location (2, 3, 4, 5) on the basis of a model of the process or at least one subprocess thereof and compares it with the measured value transmitted by the sensor at this measurement location (2, 3, 4, 5) and infers a sensor fault at this measurement location (2, 3, 4, 5) if a maximum deviation between the calculated parameter value and the transmitted measured value is exceeded.

2. Method according to Claim 1, **characterized in that,** in the event of a sensor fault, the calculated parameter value for this measurement location (2, 3, 4, 5) is transmitted to the control or regulating unit (6) instead of the measured value from this sensor.

3. Method according to Claim 1, **characterized in that,** in the event of a sensor fault, a measured value from this sensor which has been changed by a predefined correction value is transmitted to the control or regulating unit (6).

4. Method for monitoring a plurality of sensors which are arranged at different measurement locations (2, 3, 4, 5) in a process for producing and/or finishing a paper, cardboard, tissue or other fibrous web and/or for forming and/or treating a fibrous suspension (1) suitable for producing the fibrous web, in order to measure the same parameter of the fibrous suspension (1) and/or of the fibrous web and to transmit it to a control or regulating unit (6) of the process or a subprocess thereof, **characterized in that** a monitoring unit (7) calculates the difference value between the parameter values at two measurement locations (2, 3, 4, 5) on the basis of a model of the process or at least one subprocess thereof and compares it with the difference value between the measured values transmitted by the sensors at the corresponding measurement locations (2, 3, 4, 5) and infers a sensor fault at at least one of these two measurement locations (2, 3, 4, 5) if a maximum deviation between the calculated difference values and the determined difference values is exceeded.

5. Method according to Claim 4, **characterized in that,** in the event of a sensor fault, the calculated parameter values for both measurement locations (2, 3, 4, 5) are transmitted to the control or regulating unit (6) instead of the measured values from the two sensors, between which it is determined that the maximum deviation has been exceeded.

6. Method according to Claim 4, **characterized in that,** in the event of a sensor fault, the monitoring unit (7) compares at least one of the two affected measurement locations (2, 3, 4, 5) with in each case at least one further measurement location (2, 3, 4, 5) of the parameter with regard to the difference values between the calculated parameter values and the transmitted measured values.

7. Method according to Claim 6, **characterized in that,** in the case of a measurement location (2, 3, 4, 5) at which a sensor fault is identified with respect to at least two measurement points (2, 3, 4, 5), the calculated parameter value for this measurement point (2, 3, 4, 5) is transmitted to the control or regulating unit (6) instead of the measured value from this sensor.

8. Method according to Claim 6 or 7, **characterized in that,** in the case of a measurement location (2, 3, 4, 5) at which a sensor fault is identified with respect to only one of a plurality of measurement locations (2, 3, 4, 5), the measured value from the sensor at this measurement location (2, 3, 4, 5) is transmitted to the control or regulating unit (6).

9. Method according to one of the preceding claims, **characterized in that** the parameter is formed by the whiteness or the ash content of the fibrous suspension (1) and/or of the fibrous web.

10. Method according to one of the preceding claims, **characterized in that** the process comprises forming and/or treating a fibrous suspension (1).

11. Method according to Claim 9 or 10, **characterized in that** the subprocess comprises at least one flotation stage (8).

12. Method according to Claim 11, **characterized in that** a measurement location (2, 3, 4, 5) is present at least before and/or after a flotation stage, preferably all flotation stages (8).

13. Method according to Claim 11 or 12, **characterized in that** a separate control loop is assigned to at least one flotation stage, preferably to all flotation stages (8) .

14. Method according to Claim 13, **characterized in that** the control loop of a flotation stage (8) with a measurement location (2, 3, 4, 5) arranged before and after the flotation stage acts as a basis for the model of this subprocess and provides the difference value between the calculated parameter values of these measurement locations (2, 3, 4, 5).

15. Method according to one of Claims 10 to 14, **characterized in that,** in cases in which there are no subprocesses which changed the parameter between two measurement locations (2, 3, 4, 5), the model takes into account the residence time of the fibrous suspension (1) between the two measurement locations (2, 3, 4, 5).

16. Method according to one of the preceding claims, **characterized in that** the model is based on empirical values, particularly of other installations, and/or data relating to the activation and/or the history of the installation.

17. Method according to one of the preceding claims, **characterized in that** the model is in the form of a self-learning system.

## Revendications

1. Procédé de surveillance d'un capteur, lequel est disposé à un point de mesure (2, 3, 4, 5) dans un processus destiné à la production et/ou la transformation d'une bande de papier, de carton, de tissu ou d'une autre matière fibreuse ou destiné à la formation et/ou au traitement d'une suspension de matière fibreuse (1) appropriée pour la production d'une bande de matière fibreuse afin de mesurer un paramètre de la suspension de matière fibreuse (1) ou de la bande de matière fibreuse et le communiquer à une unité de commande ou de régulation (6) du processus ou d'un processus partiel de celui-ci, **caractérisé en ce qu'**une unité de surveillance (7) calcule la valeur de paramètre à un point de mesure (2, 3, 4, 5) sur la base d'un modèle du processus ou d'au moins un processus partiel de celui-ci et la compare à la valeur mesurée communiquée par le capteur de ce point de mesure (2, 3, 4, 5) et, en cas de dépassement d'un écart maximal entre la valeur de paramètre calculée et la valeur mesurée communiquée, conclut à un défaut de capteur à ce point de mesure (2, 3, 4, 5).

2. Procédé selon la revendication 1, **caractérisé en ce que** dans le cas d'un défaut de capteur, la valeur de paramètre calculée pour ce point de mesure (2, 3, 4, 5) est communiquée à l'unité de commande ou de régulation (6) à la place de la valeur mesurée de ce capteur.

3. Procédé selon la revendication 1, **caractérisé en ce que** dans le cas d'un défaut de capteur, une valeur mesurée de ce capteur modifiée par une valeur de correction prédéfinie est communiquée à l'unité de commande ou de régulation (6).

4. Procédé de surveillance de plusieurs capteurs, lesquels sont disposés à différents points de mesure (2, 3, 4, 5) dans un processus destiné à la production et/ou la transformation d'une bande de papier, de carton, de tissu ou d'une autre matière fibreuse ou destiné à la formation et/ou au traitement d'une suspension de matière fibreuse (1) appropriée pour la production d'une bande de matière fibreuse afin de mesurer le même paramètre de la suspension de matière fibreuse (1) ou de la bande de matière fibreuse et le communiquer à une unité de commande ou de régulation (6) du processus ou d'un processus partiel de celui-ci, **caractérisé en ce qu'**une unité de surveillance (7) calcule la valeur de la différence entre les valeurs de paramètre à deux points de mesure (2, 3, 4, 5) sur la base d'un modèle du processus ou d'au moins un processus partiel de celui-ci et la compare à la valeur de la différence entre les valeurs mesurées communiquées par les capteurs des points de mesure (2, 3, 4, 5) correspondants et, en cas de dépassement d'un écart maximal entre les valeurs de différence calculées et déterminées, conclut à un défaut de capteur à au moins l'un de ces deux points de mesure (2, 3, 4, 5).

5. Procédé selon la revendication 4, **caractérisé en ce que** dans le cas d'un défaut de capteur, les valeurs de paramètre calculées pour les deux points de mesure (2, 3, 4, 5) sont communiquées à l'unité de commande ou de régulation (6) à la place des valeurs mesurées des deux capteurs entre lesquels est constaté un dépassement de l'écart maximal.

6. Procédé selon la revendication 4, **caractérisé en ce que** l'unité de surveillance (7), dans le cas d'un défaut de capteur, compare au moins l'un des deux points de mesure (2, 3, 4, 5) concernés avec respectivement un autre point de mesure (2, 3, 4, 5) du paramètre pour ce qui concerne les valeurs de différence entre les valeurs de paramètre calculées et les valeurs mesurées communiquées.

7. Procédé selon la revendication 6, **caractérisé en ce que** dans le cas d'un point de mesure (2, 3, 4, 5) au niveau duquel un défaut de capteur a été reconnu en référence à au moins deux points de mesure (2, 3, 4, 5), la valeur de paramètre calculée pour ce point de mesure (2, 3, 4, 5) est communiquée à l'unité de commande ou de régulation (6) à la place de la valeur mesurée de ce capteur.

8. Procédé selon la revendication 6 ou 7, **caractérisé en ce que** dans le cas d'un point de mesure (2, 3, 4, 5) au niveau duquel un défaut de capteur a été reconnu en référence à un seul parmi plusieurs points de mesure (2, 3, 4, 5), la valeur mesurée du capteur de ce point de mesure (2, 3, 4, 5) est communiquée à l'unité de commande ou de régulation (6).

9. Procédé selon l'une des revendications précédentes, **caractérisé en ce que** le paramètre est formé par la blancheur ou la teneur en cendres de la suspension de matière fibreuse (1) et/ou de la bande de matière fibreuse.

10. Procédé selon l'une des revendications précédentes, **caractérisé en ce que** le processus comprend la formation et/ou le traitement d'une suspension de matière fibreuse (1).

11. Procédé selon la revendication 9 ou 10, **caractérisé en ce que** le processus partiel contient au moins un étage de flottation (8).

12. Procédé selon la revendication 11, **caractérisé en ce qu'**un point de mesure (2, 3, 4, 5) est présent au moins avant et/ou après un, de préférence tous les étages de flottation (8).

13. Procédé selon la revendication 11 ou 12, **caractérisé en ce qu'**un circuit de régulation est associé à au moins un, de préférence à tous les étages de flottation (8).

14. Procédé selon la revendication 13, **caractérisé en ce que** le circuit de régulation d'un étage de flottation (8), avec un point de mesure (2, 3, 4, 5) disposé avant celui-ci et après celui-ci, fait office de base pour le modèle de ce processus partiel et délivre la valeur de différence entre les valeurs de paramètre mesurées de ces points de mesure (2, 3, 4, 5).

15. Procédé selon l'une des revendications 10 à 14, **caractérisé en ce que** dans les cas dans lesquels aucun processus partiel qui modifie le paramètre ne se trouve entre deux points de mesure (2, 3, 4, 5), le modèle prend en compte le temps de séjour de la suspension de matière fibreuse (1) entre les deux points de mesure (2, 3, 4, 5) .

16. Procédé selon l'une des revendications précédentes, **caractérisé en ce que** le modèle se base sur des valeurs empiriques, notamment d'autres installations, et/ou des données de la mise en service et/ou de l'historique de l'installation.

17. Procédé selon l'une des revendications précédentes, **caractérisé en ce que** le modèle est réalisé sous la forme d'un système à auto-apprentissage.
